# EUROPÄISCHE PATENTSCHRIFT

(11) **EP 1 009 731 B1**
(45) Veröffentlichungstag und Bekanntmachung des Hinweises auf die Patenterteilung: **09.11.2005**
(21) Anmeldenummer: 98946429.2
(22) Anmeldetag: 26.08.1998
(51) Int. Cl.: C07C 209/10, H01B 1/12

(54) **VERFAHREN ZUR HERSTELLUNG VON ARYLOLIGOAMINEN**
METHOD FOR PRODUCING ARYLOLIGOAMINES
PROCEDE DE PRODUCTION D'ARYLOLIGOAMINES

(30) Priorität: 05.09.1997 DE 19738860
(43) Veröffentlichungstag der Anmeldung: 21.06.2000
(73) Patentinhaber: Covion Organic Semiconductors GmbH, 65926 Frankfurt (DE)
(72) Erfinder: SPREITZER, Hubert, D-65929 Frankfurt am Main (DE); KREUDER, Willi, D-55126 Mainz (DE); BECKER, Heinrich, D-61479 Glashütten (DE); NEUMANN, Ute, D-65795 Hattersheim (DE)
(74) Vertreter: Luderschmidt, Schüler & Partner GbR
(86) Internationale Anmeldenummer: PCT/EP1998/005398
(87) Internationale Veröffentlichungsnummer: WO 1999/012888

(56) Entgegenhaltungen:
- EP-A- 0 611 148
- EP-A- 0 802 173
- EP-A- 0 846 676
- US-A- 5 576 460
- BARAÑANO ET AL.: "Nickel and Palladium-Catalyzed Cross-Couplings that Form Carbon-Heteroatom and Carbon-element Bonds" CURRENT ORGANIC CHEMISTRY, Bd. 1, Nr. 3, 1997, Seiten 287-305, XP002085730

## Beschreibung

Aryl- und Heteroarylderivate, welche mehrere Amineinheiten aufweisen (im folgenden Aryloligoamine), sind seit längerem in verschiedenartigen Anwendungen im Einsatz. So können sie beispielsweise als Lochleiter in der Xerographie (siehe z.B. P. M. Borsenberger, D. S. Weiss, Organic Photoreceptors for Imaging Systems, Marcel Dekker, Inc.), in organischen Elektrolumineszenzvorrichtungen (siehe. z. B. J. Kido, Bull. Electrochem. 1994, 10, 1-13; DE-A- 197 11 714) und farbstoffsensibilisierten photovoltaischen Zellen (siehe z.B. DE-A 197 11 714) Verwendung finden.

Als besonders interessant haben sich dabei unter anderem Derivate des Spirobifluorens erwiesen (siehe z.B. DE-A 19711714).

Aryloligoamine werden in der Regel über Varianten der Ullmann-Reaktion (J. March. Adv. Org. Chem. 4th Ed., S. 665, John Wiley & Sons, New York 1992) aufgebaut. So wird die Darstellung von beispielsweise 4,4',4"-Tris(N,N-diphenylamino)triphenylamin (Shirota et al., Chem.Lett. 1989, 1145-1148) und Tris-(4-phenoxazin-10-yl-phenyl)-amin (Higuchi et al., Mol.Cryst.Liq.Cryst. 1994, 242, 127-134) Ober diese Route beschrieben. Auch zur Herstellung von Lochleitern auf Basis von Spiroverbindungen, wie N,N,N',N',N",N",N"',N"'-Ociaphenylspiro-9,9'-bifluoren-2,2',7,7'-tetramin (Salbeck et al., Book of Abstracts, 213^{th} ACS National Meeting, San Francisco 1997, 199) ist diese Reaktion beschrieben.

Allgemein gilt, daß die Herstellung von Aryloligoaminen gegenüber der von Arylmonoaminen erhöhte Schwierigkeiten mit sich bringt. So ist z.B. bekannt, daß Ullmann-Reaktionen unter optimalen Bedingungen mit ca. 80% Ausbeute durchgeführt werden können. Wendet man dies auf eine Tri- oder Tetrakupplungsreaktion an, so kommt man bereits theoretisch nur noch auf 51 bzw. 41% Ausbeute, was zum einen die Wirtschaftlichkeit beeinträchtigt, zum anderen die Reinigung der Produkte erschwert.

Es wurde nun überraschend gefunden, daß sich Aryloligoamine einfach und in guten Ausbeuten durch direkte Kupplung eines primären oder sekundären Amins mit einem aktivierten Aromaten in Gegenwart einer Base, einer Palladiumkomponente und eines Phosphanliganden herstellen lassen.

Ein ähnliches Verfahren ist zwar aus der US-A 5,576,460 bekannt, es ist dort allerdings ausschließlich zur Darstellung von Arylmonoaminen beschrieben. Zudem wird im einzigen Beispiel eine Ausbeute von lediglich 75 % erhalten, wodurch der Fachmann zu dem Schluß kommt, daß eine solche Methode zum Aufbau von Aryloligoaminen weniger geeignet ist als die Ullmann-Reaktion.

Aus EP-A-0 802 173 ist ein Verfahren zur Herstellung von heterocyclischen aromatischen Aminen und Arylaminen unter Einsatz von Katalysatorsystemen aus Palladium-Verbindungen und verschiedene Phosphan liganden bekannt.

Gegenstand der Erfindung ist daher ein Verfahren zur Herstellung von Aryloligoaminen, gemäß Anspruch 1.

Aryloligoamin bedeutet im Sinne der Erfindung eine Verbindung, die mindestens zwei an aromatische Gruppen gebundene Amineinheiten enthält.

Vorzugsweise haben die Symbole und Indizes in der Formel (I) in Anspruch 1 folgende Bedeutungen:
- R: ist gleich oder verschieden NO₂, CN, F, eine unverzweigte oder verzweigte Alkylgruppe mit 1 bis 22 C-Atomen.
- X: Cl, Br, I, Tosylat;
- m: 3 ≤ m ≤ y;

Besonders bevorzugt haben die Symbole und Indizes in der Formel (I) in Anspruch 1 folgende Bedeutungen:
- A: ist Benzol, Naphthalin, Anthracen, Pyren, Triphenylen, Biphenyl, Fluoren, Terphenyl, 1,3,5-Triphenylbenzol, Spiro-9,9'-bifluoren, 2,2',7,7'-Tetraphenylspiro-9,9'-bifluoren, 2,2',7,7'-Tetra(4'-biphenylyl)spiro-9,9'-biphenyl, 2,4,7,2'4',7'-Hexaphenylspiro-9,9'-bifluoren und 2,4,7,2',4',7'-Hexa(4'-biphenylyl)spiro-9,9'-bifluoren oder ein anderes Oligophenylen-substituiertes Derivat des Spiro-9,9'-bifluorens;
- R: ist gleich oder verschieden NO₂, CN, F, eine unverzweigte oder verzweigte Alkylgruppe mit 1 bis 22 C-Atomen.
- X: ist Br, I ;
- m: ist 4, 5 oder 6 und
- n: ist 0, 1, 2, 3, 4, 5, 6.

Insbesondere bevorzugte aktivierte Aromaten der Formel (I) sind solche der Formel (Ia) 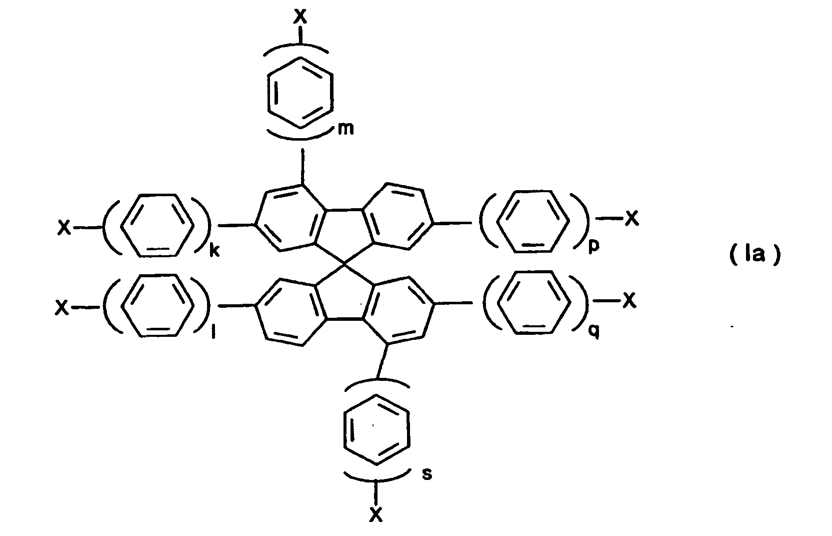 wobei
- X: gleich oder verschieden, Br, I oder H und
- k,l,p,q,r,s: 0, 1, 2, 3, 4
bedeuten, mit der Maßgabe, daß mindestens zwei, vorzugsweise mindestens vier, der Reste X Br oder I sind.

Bevorzugte Aminkomponenten sind solche der Formel (II),

H-NR⁷R⁸ (II)

wobei die Symbole folgende Bedeutungen haben:
R⁷, R⁸ sind gleich oder verschieden
   a) H;
   b) eine geradkettige, verzweigte oder cyclische Alkylgruppe mit 1 bis 22 C-Atomen, wobei eine oder mehrere CH₂-Gruppen durch -O- , -S- , -CO- , -O-CO-, -CO-O-, -O-CO-O-, -CR¹=CR², -C≡C-, SiR³R⁴, C₄-C₁₀-Aryldiyl, C₄-C₁₀-Heteroaryldiyl, Cyclohexylen, -NR⁵-, wobei Heteroatome nicht direkt aneinander geknüpft sein dürfen, und wobei ein oder mehrere H-Atome durch F, Cl, Br ersetzt sein können; wobei R¹ bis R⁵ die in der Formel (I) angegebenen Bedeutungen haben;
   c) eine C₄-C₁₂-Aryl- oder Heteroarylgruppe die durch einen oder mehrere Reste R substituiert sein kann, wobei R gleich oder verschieden die in der Formel (I) angegebenen Bedeutungen hat.

Endprodukte des erfindungsgemäßen Verfahrens sowie deren Bevorzugungen ergeben sich aus den Edukten und deren Bevorzugungen.

Die Edukte des Verfahrens sind prinzipiell bekannt und entweder kommerziell erhältlich oder nach bekannten, dem Fachmann geläufigen Methoden, wie sie beispielsweise in Houben-Weyl, Methoden der Organischen Chemie, Georg Thieme Verlag, Stuttgart, aufgeführt sind, herstellbar.

Das Verhältnis der Edukte ist nicht kritisch und kann daher in weiterem Rahmen variiert werden, bevorzugt ist ein Verhältnis aktivierte Gruppe zu Amin von 1: 0,8 bis 2, besonders bevorzugt 1: 1 bis 1,5.

Erfindungsgemäß werden die Ausgangsverbindungen, Amin und aktivierter Aromat, in einer Kupplungsreaktion zu einem Aryloligoamin umgesetzt.
Zur Durchführung der Reaktion werden das Amin, der aktivierte Aromat, eine Base und katalytische Mengen eines Phosphanliganden enthaltenden Palladiumkatalysators bzw. ein Palladiumsalz und ein Phosphan in einem Lösungsmittel aufgenommen und bei einer Temperatur von 0°C bis 150°C, bevorzugt bei 30°C bis 140° C, besonders bevorzugt bei 50°C bis 120°C, insbesonders bevorzugt bei 80°C bis 120°C für einen Zeitraum von 1 h bis 200 h, bevorzugt 5 h bis 100 h, besonders bevorzugt 10 h bis 80 h, umgesetzt. Die Aufarbeitung erfolgt nach an sich bekannten, dem Fachmann geläufigen Methoden, beispielsweise durch Hydrolyse, Ausrühren, Phasenseparation und Abziehen des Lösungsmittels. Bevorzugt wird auch die Palladium-Komponente durch Ausrühren mit einem Komplexbildner entfernt. Hier eignet sich unter anderem Cyanid, Thiocyanat u.ä..
Das Rohprodukt kann dann nach dem Fachmann bekannten und dem jeweiligen Produkt angemessenen Methoden, z.B. durch Umkristallisation, Destillation, Sublimation, Zonenschmelzen, Schmelzkristallisation oder Chromatographie, aufgereinigt werden.

Das erfindungsgemäße Verfahren wird im allgemeinen in einem Lösungsmittel durchgeführt, wobei auch ein Überschuß der Base oder eines Edukts, vorzugsweise der Aminkomponente, als solches Lösungsmittel dienen kann.

Bevorzugt ist die Verwendung eines oder mehrerer organischer Lösungsmittel oder einer Mischung aus Wasser und einem oder mehreren organischen Lösungsmitteln, wobei in diesem Fall vorzugsweise mindestens eines der organischen Lösungsmittel wasserunlöslich sein sollte.
Bevorzugte organische Lösungsmittel sind Ether, z. B. Diethylether, Dimethoxymethan, Diethylenglykoldimethylether, Tetrahydrofuran, Dioxan, Dioxolan, Diisopropylether, tert.-Butylmethylether, Kohlenwasserstoffe, z. B. Hexan, iso-Hexan, Heptan, Cyclohexan,Toluol, Xylol, Alkohole, z. B. Methanol, Ethanol, 1-Propanol, 2-Propanol, Ethylenglykol, 1-Butanol, 2-Butanol, tert.-Butanol, Ketone, z. B. Aceton, Ethylmethylketon, iso-Butylmethylketon, Amide, z.B. Dimethylformamid, Dimethylacetamid, N-Methylpyrrolidon, Nitrile, z.B. Acetonitril, Propionitril, Butyronitril, und Mischungen derselben.

Besonders bevorzugte organische Lösungsmittel sind Ether, wie Dimethoxyethan, Diethylenglykoldimethylether, Tetrahydrofuran, Dioxan, Diisopropylether, Kohlenwasserstoffe, wie Hexan, Heptan, Cyclohexan, Toluol, Xylol, Alkohole, wie Methanol, Ethanol, 1-Propanol, 2-Propanol, 1-Butanol, 2-Butanol, tert.-Butanol, Ethylenglykol, Ketone, wie Methylethylketon, iso-Butylmethylketon, Amide, wie Dimethylformamid, Dimethylacetamid, N-Methylpyrrolidon, und Mischungen derselben.

Ganz besonders bevorzugte organische Lösungsmittel sind Ether, z. B. Dimethoxyethan, Tetrahydrofuran, Dioxan, Kohlenwasserstoffe, z. B. Cyclohexan, Toluol, Xylol, Alkohole, z. B. Ethanol, 1-Propanol, 2-Propanol, 1-Butanol, tert.-Butanol und Mischungen derselben.

Beispiele für die Verwendung von Wasser und organischen Lösungsmitteln sind Mischungen aus Wasser und Toluol sowie Wasser, Toluol und Tetrahydrofuran.

Basen, die bei dem erfindungsgemäßen Verfahren vorzugsweise Verwendung finden, sind Alkali- und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkoholate, sowie primäre, sekundäre und tertiäre Amine.
Dies bedeutet, daß die eingesetzte Aminkomponente, einen entsprechenden Überschuß vorausgesetzt, auch als Base wirken kann.
Besonders bevorzugt sind Alkali- und Erdalkalimetallhydroxide, Alkali- und Erdalkalimetallcarbonate und Alkali- und Erdalkalimetallalkoholate.
Insbesondere bevorzugt sind Alkalimetallhydroxide, wie Natriumhydroxid und Kaliumhydroxid, sowie Alkali- und Erdalkalimetallalkoholate, wie Natriumethanolat und Natrium- oder Kalium-tert.-butanolat.

Die Base wird bei dem erfindungsgemäßen Verfahren bevorzugt mit einem Anteil von 50 bis 500 Mol-%, besonders bevorzugt 50 bis 250 Mol-%, ganz besonders bevorzugt 75 bis 200 Mol-%, insbesondere 90 bis 120 Mol-%, bezogen auf vorhandene Mole N-H, eingesetzt.

Die Palladiumkomponente enthält Palladiummetall oder eine Palladium (0) oder (II) Verbindung.

Palladiumkomponente und Phosphanligand können als Komplex, z.B. als das u. a. bevorzugte Pd(PPh₃)₄, oder getrennt eingesetzt werden.

Als Palladiumkomponente eignen sich beispielsweise Palladiumverbindungen, wie Palladiumketonate, Palladiumacetylacetonate, Nitrilpalladiumhalogenide, Olefinpalladiumhalogenide, Palladiumhalogenide, Allylpalladiumhalogenide und Palladiumbiscarboxylate, bevorzugt Palladiumketonate, Palladiumacetylacetonate, bis-η²-Olefinpalladiumdihalogenide, Palladium(II)halogenide, η³-Allylpalladiumhaiogenid Dimere und Palladiumbiscarboxylate, ganz besonders bevorzugt Bis(dibenzylidenaceton)palladium(0) [Pd(dba)₂)], Pd(dba)₂ CHCl₃, Palladiumbisacetylacetonat, Bis(benzonitril)palladiumdichlorid, PdCl₂, Na₂PdCl₄, Dichlorobis(dimethytsulfoxid)palladium(II), Bis(acetonitril)palladiumdichlorid, Palladium-II-acetat, Palladium-II-propionat, Palladium-II-butanoat und (1c,5c-Cyclooctadien)palladiumdichlorid.

Ebenso als Palladiumkomponente dienen kann Palladium in metallischer Form, im folgenden nur Palladium genannt, vorzugsweise Palladium in pulverisierter Form oder auf einem Trägermaterial, z.B. Palladium auf Aktivkohle, Palladium auf Aluminiumoxid, Palladium auf Bariumcarbonat, Palladium auf Bariumsulfat, Palladium auf Aluminiumsilikaten, wie Montmorillonit, Palladium auf SiO₂ und Palladium auf Calciumcarbonat, jeweils mit einem Palladiumgehalt von 0,5 bis 10 Gew.-%. Besonders bevorzugt sind Palladium in pulverisierter Form, Palladium auf Aktivkohle, Palladium auf Barium- und/oder Calciumcarbonat und Palladium auf Bariumsulfat, jeweils mit einem Palladiumgehalt von 0,5 bis 10 Gew.-% insbesondere bevorzugt ist Palladium auf Aktivkohle mit einem Palladiumgehalt von 5 oder 10 Gew.-%

Die Palladiumkomponente wird bei dem erfindungsgemäßen Verfahren mit einem Anteil von 0,01 bis 10 Mol-%, bevorzugt 0.05 bis 5 Mol-%, besonders bevorzugt 0,1 bis 3 Mol-%, insbesondere bevorzugt 0,1 bis 1,5 Mol-%, bezogen auf vorhandene N-H-Gruppen, eingesetzt.

Für das erfindungsgemäße Verfahren geeignete Phosphanliganden sind beispielsweise Tricycloalkylphosphane ausgenommen Tricyclohexylphosphan, Triarylphosphane, wobei die drei Substituenten am Phosphor gleich oder verschieden, chiral oder achiral sein können und wobei einer oder mehrere der Liganden die Phosphorgruppen mehrerer Phosphane verknüpfen können und wobei ein Teil dieser Verknüpfung auch ein oder mehrere Metallatome sein können.
Beispiele für im Rahmen des erfindungsgemäßen Verfahrens verwendbare Phosphane sind Triphenylphosphan, Tritolylphosphan, Tris-(4-dimethylaminophenyl)-phosphan, Bis(diphenylphosphano)methan, 1,2-Bis(diphenylphosphano)ethan, 1,3-Bis(diphenylphosphano)propan und 1,1'-Bis(diphenylphosphano)-ferrocen.

Ganz besonders bevorzugt sind Triphenylphosphan, Tris(o-tolyl)phosphan, 1,2-Bis(diphenylphosphano)ethan, 1,3-Bis(diphenylphosphano)propan und 1,1'-Bis(diphenylphosphano)-ferrocen, insbesondere Triphenylphosphan und Tris(o-tolyl)phosphan.
Für das erfindungsgemäße Verfahren weiterhin geeignet sind wasserlösliche Phosphanliganden, die beispielsweise Sulfonsäuresalz- und/oder Sulfonsäurereste und/oder Carbonsäuresalz- und/oder Carbonsäurereste und/oder
Phosphonsäuresalz und/oder Phosphonsäurereste und/oder Phosphoniumgruppen und/oder Peralkylammoniumgruppen und/oder Hydroxygruppen und/oder Polyethergruppen mit geeigneter Kettenlänge enthalten.

Bevorzugte Klassen von wasserlöslichen Phosphanliganden sind mit den obigen Gruppen substituierte, Tricycloalkylphosphane, ausgenommen Tricyclohexylphosphan, Triarylphosphane, Dialkylarylphosphane, Alkyldiarylphosphane und Heteroarylphosphane wie Tripyridylphosphan und Trifurylphosphan, wobei die drei Substituenten am Phosphor gleich oder verschieden, chiral oder achiral sein können und wobei einer oder mehrere der Liganden die Phosphorgruppen mehrerer Phosphane verknüpfen können und wobei ein Teil dieser Verknüpfung auch ein oder mehrere Metallatome sein können.

Der Phosphanligand wird bei dem erfindungsgemäßen Verfahren mit einem Anteil von 0,1 bis 20 Mol %, bevorzugt 0,2 bis 15 Mol %, besonders bevorzugt 0,5 bis 10 Mol %, insbesonders bevorzugt 1 bis 6 Mol %, bezogen auf vorhandene N-H-Gruppen, eingesetzt.
Es können gegebenenfalls auch Mischungen zweier oder mehrerer verschiedener Phosphanliganden eingesetzt werden.

Die Produkte des erfindungsgemäßen Verfahrens eignen sich z.B. zur Verwendung in Elektrolumineszenzvorrichtungen, farbstoffsensibilisierten photovoltaischen Zellen oder in der Xerographie, beispielsweise als Lochleitermaterialien.

Die Erfindung wird durch die nachfolgenden Beispiele näher erläutert, ohne sie dadurch einschränken zu wollen.

### Beispiel 1:

### Herstellung von 2,2',7,7'-Tetrakis(N,N-diphenylamino)-spiro-9,9'-bifluoren

2,2',7,7'-Tetrabrom-9,9'-spirobifluoren (20,86 g, 33 mmol) und Diphenylamin (25,38 g, 150 mmol) wurden in Toluol (400 ml) vorgelegt und die Lösung mit N₂ gesättigt. Anschließend gab man Pd(OAc)₂ (311 mg, 1,5 mmol), P(o-Tolyl)₃ (0,913 g, 3 mmol) und die Base (NaO^{t}Bu, 20,18 g, 210 mmol) zu. Die Lösung färbte sich sofort dunkelgrün. Es wurde für 72 Stunden unter Rückfluß gerührt.
Nach Abkühlen des Ansatzes auf Raumtemperatur wurde NaCN-Lösung zugeben (ca 2 g NaCN in 200 ml Wasser) und ca. 60 min gerührt. Anschließend wurde dreimal mit Wasser gewaschen. Die organische Phase wurde über Na₂SO₄ getrocknet und das Lösungsmittel anschließend abgezogen. Man erhielt hellbraune Kristalle.
Diese wurden in 100 ml Dioxan und 5 ml Hydrazinhydrat umkristallisiert. Dieser Vorgang wurde einmal wiederholt. Schließlich wurden die erhaltenen Kristalle mehrfach mit Hexan ausgerührt und im Vakuum bei 60°C getrocknet.
Man erhielt 19.3 g (59%) farbloses Pulver, welches gemäß HPLC eine Reinheit von größer 99.7% aufwies.
¹H NMR (CDCl₃, NH₂NH₂*H₂O): δ [ppm] = 7.45 (d, 4H, H-4, J = 8 Hz), 7.19 (m, 16 H, H-3'), 6.97 (m, 24 H, H-2', H-4'), 6.92 (dd, 4 H, H-3, J₁ = 2, J₂ = 8 Hz), 6.69 (d, 4 H, H-1, J = 2 Hz).

Die Verwendung von DPPF (Bis(diphenylphosphino)ferrocen) als Ligand führte zu einer Ausbeuteverschlechterung (45%; Reinheit: >99.9%), die Verwendung von BINAP (2,2'-Bis(diphenylphosphino)-1,1'-binaphthyl) hingegen zu einer leichten Ausbeutesteigerung (62%; Reinheit: >99.7%).

### Beispiel 2:

### Herstellung von 2,2',7,7'-Tetrakis(N,N-di-4-methoxyphenylamino)-spiro-9,9'-bifluoren

2,2',7,7'-Tetrabrom-9,9'-spirobifluoren (13.9 g, 22 mmol) und 4,4'-Dimethoxydiphenylamin (22.98 g, 100 mmol) wurden in Toluol (250 ml) gelöst und die Lösung mit N₂ gesättigt. Die Lösung verfärbte sich braun. Anschließend gab man Pd(OAc)₂ (223 mg, 1 mmol) und P(o-Tolyl)₃ (608 mg, 2 mmol) zu. NaO'Bu (13.5 g, 140 mmol) wurde als Lösung in Dioxan (in 150ml) zugetropft. Die Lösung färbte sich beim Zutropfen langsam dunkelbraun. Die Lösung wurde unter Schutzgas für 72 Stunden am Rückfluß gerührt.
Nach Abkühlen auf Raumtemperatur wurde mit H₂O hydrolysiert, die Wasserphase abgetrennt, mit NaCN-Lösung (3 g NaCN in 300ml Wasser) 2h ausgerührt und anschließend erneut die Wasserphase abgetrennt. Die organische Phase wurde filtriert und das Lösungsmittel abgezogen. Man erhielt ein schwarzes Öl , das mit der Zeit erstarrte. Es wurde in 50 ml Dioxan heiß gelöst in der Wärme mit 50 ml Ethanol versetzt und unter Rühren abgekühlt. Dabei fiel ein gelb-grüner Feststoff aus. Dieser wurde abgesaugt und mehrmals mit kleinen Portionen (je 2 ml) Dioxan nachgewaschen; bis die Farbe hellgelb war. Anschließend wurde nochmals aus ganz wenig Dioxan umkristallisiert (Zusatz von einigen Tropfen Hydrazinhydrat).

Es wurden 13.9 g (52%) fast farbloses Pulver erhalten, welches laut ¹H-NMR eine Reinheit von 99% aufwies.
¹H NMR (CDCl₃, NH₂NH₂*H₂O): δ [ppm] = 7.36 (d, 4H, H-4, J = 8 Hz), 6.90 + 6.75 (AA'BB', jeweils 16 H, H-2', H-3'), 6.79 (dd, 4 H, H-3, J₁ = 2, J₂ = 8 Hz), 6.55 (d, 4 H, H-1, J = 2 Hz), 3.77 (s, 24 H, OMe).

### Beispiel 3:

### Herstellung von 2,2',7,7'-Tetrakis(N-phenothiazinyl)-spiro-9,9'-bifluoren

Die Umsetzung wurde analog zu Beispiel 2 durchgeführt. Bei der Aufarbeitung wurde nach Ausrühren mit NaCN-Lösung der entstandene Feststoff abgesaugt. Der erhaltene Feststoff wurde zunächst mit Aceton : Wasser : Hydrazinhydrat (70:15:3) ca. 1 Stunde ausgekocht und schließlich aus Chloroform : Ethanol (1:1) umkristallisiert. Man erhielt das reine Produkt (laut ¹H-NMR Reinheit >99%) als farbloses Pulver. 47% Ausbeute.
¹H NMR (CDCl₃, NH₂NH₂*H₂O): δ [ppm] = 8.06 (d, 4H, H-4, J = 8 Hz), 7.43 (dd, 4 H, H-3, J₁ = 2, J₂ = 8 Hz), 7.08 (d, 4 H, H-1, J = 2 Hz), 6.96 (dd, 4 H, H-4', J₁ = 1.5, J₂ = 7.5 Hz), 6.71 (dt, 4 H, H-3', J₁ = 1.2, J₂ = 7.5 Hz), 6.47 (ddd, 4 H, H-2', J₁ = 1.5, J₂ = 7.3 Hz, J₃ = 8.3 Hz), 5.98 (dd, 4 H, H-1', J₁ = 1.0, J₂ = 8.3 Hz).

### Beispiel 4:

### Herstellung von 2,2',7,7'-Tetrakis(N,N-di-4-methylphenylamino)-spiro-9,9'-bifluoren

2,2',7,7'-Tetrabrom-9,9'-spirobifluoren und 4,4'-Dimethyldiphenylamin wurden analog zu Beispiel 2 umgesetzt. Die Aufarbeitung erfolgte ebenfalls analog.
Nach Trocknung erhielt man 51% Produkt (Reinheit: >99.5% nach NMR).
¹H NMR (CDCl₃, NH₂NH₂*H₂O): δ [ppm] = 7.39 (d, 4H, H-4, J = 8 Hz), 6.99 + 6.88 (AA'BB', jeweils 16 H, H-2', H-3'), 6.85 (dd, 4 H, H-3, J₁ = 2, J₂ = 8 Hz), 6.66 (d, 4 H, H-1, J = 2 Hz), 2.29 (s, 24 H, Me).

### Beispiel 5:

### Herstellung von 2,2',7,7'-Tetrakis(N-phenyl-N-(3-methylphenyl)amino)-spiro-9,9'-bifluoren

2,2',7,7'-Tetrabrom-9,9'-spirobifluoren und 3-Methyldiphenylamin wurden analog zu Beispiel 1 umgesetzt. Als Ligand wurde DPPF verwendet. Die Aufarbeitung erfolgte ebenfalls analog, jedoch erwies sich das Produkt als deutlich schwerer reinigbar. So wurde zunächst zweimal über eine Kieselgelsäule filtriert und anschließend zunächst aus Ethylacetat/Methanol (2:1), dann mehrfach aus Dioxan umkristallisiert. Nach Trocknung erhielt man 26% Produkt (Reinheit: >99.9% nach HPLC).
¹H NMR (CDCl₃, NH₂NH₂*H₂O): δ [ppm] = 7.43 (d, 4H, H-4, J = 8 Hz), 7.18 + 6.97 + 6.94 (AA'BB'C, 8 + 8 + 4 H, H-3', H-2', H-4'), 7.08 (t, 4 H, H-5", J = 7 Hz), 6.89 (dd, 4 H, H-3, J₁ = 2, J₂ = 8 Hz), 6.84 (s (br), 4 H, H-2"), 6.78 (m, 8 H, H-4", H-6"), 6.68 (d, 4 H, H-1, J = 2 Hz), 2.22 (s, 12 H, Me).

### Beispiel 6:

### Herstellung von 2,2',7,7'-Tetrakis(N,N-di-4-biphenylamino)-spiro-9,9'-bifluoren

2,2',7,7'-Tetrabrom-9,9'-spirobifluoren und 4,4'-Biphenylamin wurden analog zu Beispiel 2 umgesetzt. Die Aufarbeitung erfolgte ebenfalls analog.
Nach Trocknung erhielt man 31% Produkt (Reinheit >99.7% nach HPLC).
¹H NMR (CDCl₃, NH₂NH₂*H₂O): δ [ppm] = 7.53 (d, 4H, H-4, J = 8 Hz), 7.51 + 7.37 + 7.28 (AA'BB'C, 16 + 16 + 8 H, H-2", H-3", H-4"), 7.42 + 7.12 (AA'BB', jeweils 16 H, H-2', H-3'), 7.03 (dd, 4 H, H-3, J₁ = 2, J₂ = 8 Hz), 6.86 (d, 4 H, H-1, J = 2 Hz).

### Beispiel 7:

### Herstellung von 2,2',7,7'-Tetrakis(N-phenyl-N-(2-naphthyl)amino)-spiro-9,9'-bifluoren

2,2',7,7'-Tetrabrom-9,9'-spirobifluoren und 2-Naphthylphenylamin wurden analog zu Beispiel 5 umgesetzt. Die Aufarbeitung erfolgte ebenfalls analog, das Produkt erwies sich ebenfalls als schwer reinigbar. So wurde zunächst zweimal über eine Kieselgelsäule filtriert und dann mehrfach aus Dioxan/Methanol umkristallisiert.
Nach Trocknung erhielt man 35% Produkt (Reinheit: >99.7% nach HPLC).
¹H NMR (CDCl₃, NH₂NH₂*H₂O): δ [ppm] = 7.75 (dd, 4 H, H-5_{NP}, J₁ = 2, J₂ = 7 Hz), 7.67 (d, 4 H, H-4_{NP}, J = 9 Hz), 7.53 (dd, 4 H, H-8_{NP}, J₁ = 2, J₂ = 7.5 Hz), 7.44 (d, 4H, H-4, J = 8 Hz), 7.35 (m, 12 H, H-1_{NP}, H-6_{NP}, H-7_{NP}), 7.23 + 7.08 + 7.03 (AA'BB'C, 8 + 8 + 4 H, H-3_{PH}, H-2_{PH}, H-4_{PH}), 7.19 (dd, 4 H, H-3_{NP}, J₁ = 2, J₂ = 8.8 Hz), 6.95 (dd, 4 H, H-3, J₁ = 2, J₂ = 8 Hz), 6.76 (d, 4 H, H-1, J = 2 Hz).

## Patentansprüche

1. Verfahren zur Herstellung von Aryloligoaminen die mindestens zwei an aromatische Gruppen gebundene Amineinheiten enthalten, **dadurch gekennzeichnet, daß** man ein Amin mit einem aktivierten Aromaten der allgemeinen Formel (I)
(R)ₙ-A-(X)ₘ (I)
wobei die Symbole und Indizes folgende Bedeutung haben:
A ist ein aromatischer Rest mit 2 bis 200 C-Atomen, der mehrere aromatische Gruppen enthalten kann, wobei solche Gruppen dann kondensiert sind;
R ist gleich oder verschieden NO₂, CN, F, eine unverzweigte oder verzweigte Alkylgruppe mit 1 bis 22 C-Atomen, wobei eine oder mehrere CH₂-Gruppen durch -O- , -S- , -CO- , -O-CO- , -CO-O- , -O-CO-O- , -CR¹=CR² , -C≡C-, SiR³R⁴ , C₄-C₁₀-Aryldiyl, C₄-C₁₀-Heteroaryldiyl, Cyclohexylen, -NR⁵- ersetzt sein können, wobei Heteroatome nicht direkt aneinander geknüpft sein dürfen, und wobei ein oder mehrere H-Atome durch F, Cl, Br ersetzt sein können;
R¹, R² sind gleich oder verschieden H, CN, C₁-C₁₂-Alkyl, C₄-C₁₀-Aryl;
R³, R⁴ sind gleich oder verschieden C₁-C₁₂-Alkyl, C₄-C₁₀-Aryl;
R⁵ ist C₁-C₁₂-Alkyl, C₄-C₁₀-Aryl;
X ist Cl, Br, I, Mesylat, Tosylat oder C₁-C₁₂-Perfluoralkylsulfonat;
m ist eine natürliche Zahl, wobei gilt 2 ≤ m ≤ y;
n ist eine natürliche Zahl, wobei gilt 0 ≤ n ≤ y-m;
y ist die Zahl der freien Valenzen am Grundkörper A,
und einer Base in einem Temperaturbereich von 0 bis 150°C in Gegenwart einer Palladiumkomponente und eines Phosphanliganden, ausgenommen Trialkylphosphan und Tricyclohexylphosphan, umsetzt

2. Verfahren zur Herstellung von Aryloligoaminen die mindestens zwei an aromatische Gruppen gebundene Amineinheiten enthalten, **dadurch gekennzeichnet, daß** man ein Amin mit einem aktivierten Aromaten der Formel (la), 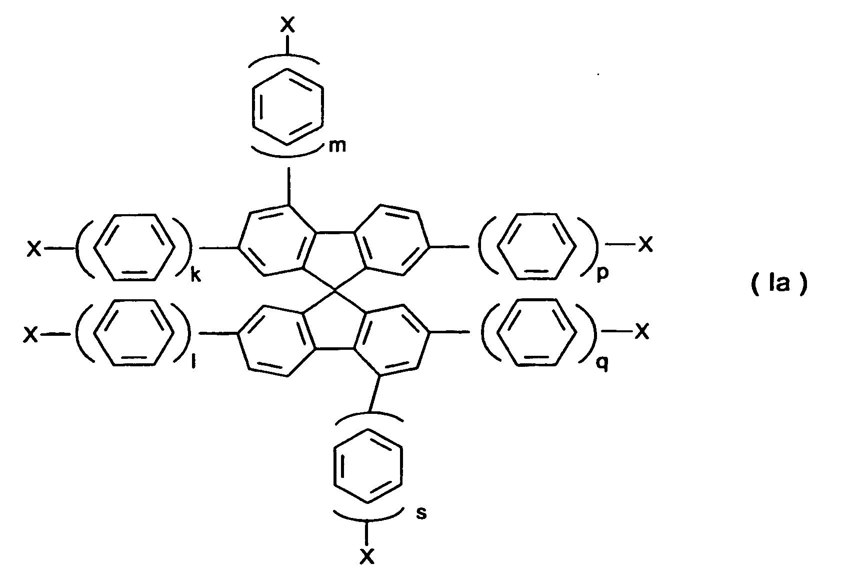 wobei
X gleich oder verschieden, Br, I oder H und
k,l,p,q,m,s 0, 1, 2, 3, 4
bedeuten, mit der Maßgabe, daß mindestens zwei der Reste X Br oder I sind, und einer Base in einem Temperaturbereich von 0 bis 150°C in Gegenwart einer Palladiumkomponente und eines Phosphanliganden umsetzt.

3. Verfahren nach einem oder mehreren der vorhergehenden Ansprüche, **dadurch gekennzeichnet, daß** man eine Aminkomponente der Formel (II) einsetzt,
H-NR⁷R⁸ (II)
wobei die Symbole folgende Bedeutungen haben:
R⁷, R⁸ sind gleich oder verschieden
a) H;
b) eine geradkettige, verzweigte oder cyclische Alkylgruppe mit 1 bis 22 C-Atomen, wobei eine oder mehrere CH₂-Gruppen durch -O-, -S-, -CO-, -O-CO-, -CO-O-, -O-CO-O-, -CR¹=CR², -C≡C-, SiR³R⁴, C₄-C₁₀-Aryldiyl , C₄-C₁₀-Heteroaryldiyl, Cyclohexylen, -NR⁵-, wobei Heteroatome nicht direkt aneinander geknüpft sein dürfen, und wobei ein oder mehrere H-Atome durch F, Cl, Br ersetzt sein können; wobei R¹ bis R⁵ die in der Formel (I) in Anspruch 1 angegebenen Bedeutungen haben;
c) eine C₄-C₁₂-Aryl- oder Heteroarylgruppe die durch einen oder mehrere Reste R substituiert sein kann, wobei R gleich oder verschieden die in der Formel (I) in Anspruch 1 angegebenen Bedeutungen hat.

4. Verfahren nach einem oder mehreren der vorhergehenden Ansprüche, **dadurch gekennzeichnet, daß** man ein Verhältnis aktivierte Gruppe am Aromaten zu Amin von 1 : 0,8 bis 2, bevorzugt 1 : 1 bis 1,5, wählt.

5. Verfahren nach einem oder mehreren der vorhergehenden Ansprüche, **dadurch gekennzeichnet, daß** man eine Base aus der Gruppe Alkali- und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkoholate und primäre, sekundäre und tertiäre Amine einsetzt.

6. Verfahren nach einem oder mehreren der vorhergehenden Ansprüche, **dadurch gekennzeichnet, daß** man als Palladiumkomponente Palladiummetall, eine Palladiumverbindung oder einen Palladiumkomplex, der einen Phosphanliganden enthält, einsetzt.

7. Verfahren nach einem oder mehreren der vorhergehenden Ansprüche, **dadurch gekennzeichnet, daß** man die Palladiumkomponente mit einem Anteil von 0,01 bis 10 Mol-%, bevorzugt 0,05 bis 5 Mol-%, besonders bevorzugt 0,1 bis 3 Mol-%, insbesondere bevorzugt 0,1 bis 1,5 Mol-%, bezogen auf vorhandene N-H-Gruppen, einsetzt.

8. Verfahren nach einem oder mehreren der vorhergehenden Ansprüche, **dadurch gekennzeichnet, daß** man einen Phosphanliganden aus der Gruppe Tricycloalkylphosphane, Triarylphosphane einsetzt, wobei die drei Substituenten am Phosphor gleich oder verschieden, chiral oder achiral sein können und wobei einer oder mehrere der Liganden die Phosphorgruppen mehrerer Phosphane verknüpfen können und wobei ein Teil dieser Verknüpfung auch ein oder mehrere Metallatome sein können.

## Claims

1. A process for preparing aryl oligoamines, comprising at least two amine units linked to an aromatic groups, which comprises reacting an amine with an activated aromatic is a compound of the formula (I),
(R)n-A-(X)m (I)
where the symbols and indices have the following meanings:
A is an aromatic and/or heteroaromatic radical which has from 2 to 200 carbon atoms and can contain a plurality of aromatic and/or heteroaromatic groups, where such groups are then fused;
R are identical or different and are each NO2, CN, F, an unbranched or branched alkyl group having from 1 to 22 carbon atoms, where one or more CH2 groups may be replaced by -0-, -S-, -CO-, -O-CO-, -CO-0-, -0-00-0-, -CR¹=CR², -C=C-, SiR³R⁴, ^{c}4-^{c}10-aryldiyl, C4-C10-heteroaryldiyl, cyclohexylene, -NR⁵-, where heteroatoms must not be directly bonded to one another, and where one or more H atoms may be replaced by F, Cl, Br;
R¹, R² are identical or different and are H, CN, C₁-C₁₂-alkyl, C₄-C₁₀-aryl
R³, R⁴ are identical or different and are C₁-C₁₂-alkyl, C₄-C₁₀-aryl;
R⁵ is C1-C12-alkyl, C4-C10-aryl;
x is Cl, Br, 1, mesylate, tosylate or C1-C12-perfluoroalkylsulfonate;
m is a natural number and 2 m <_ y;
n is a natural number and n <_ y-m;
y is the number of free valences an the parent unit A.
and a base in a temperature range 5 from 0 to 150°C in the presence of a palladium component and a phosphine ligand, excluding trialkyphosphane and tricyclohexelem phosphane.

2. The process comprising at least two amine units linked to an aromatic groups wherein the activated aromatic used is a compound of the formula (la), 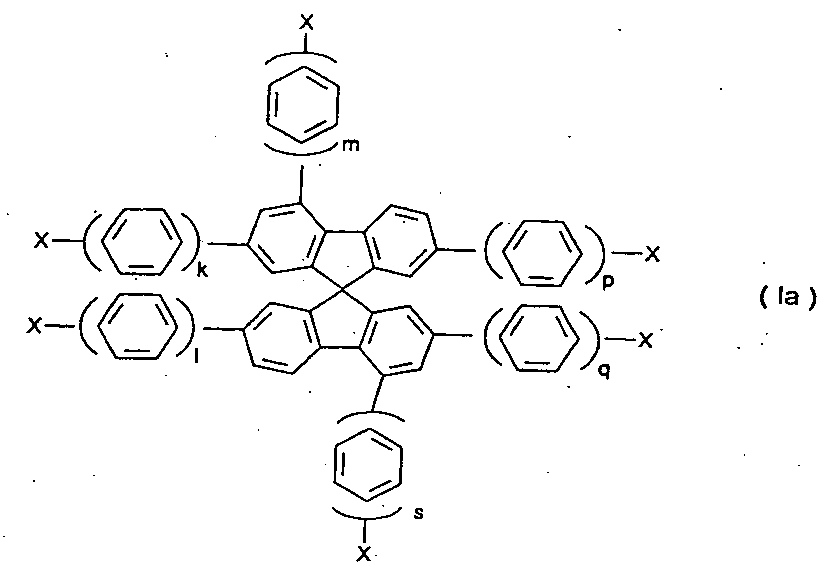 where
X are identical or different and are each Br, 1 or H and
k,l,p,q,r,s are 0, 1, 2, 3, 4,
with the proviso that at least two of the radicals X are Br or I.

3. The process as claimed in one or more of the preceding claims, wherein the amine component used is a compound of the formula (II),
H-NR⁷R⁸ (II)
where the symbols have the following meanings:
R⁷, R⁸ are identical or different and are each
a) H;
b) a straight-chain, branched or cyclic alkyl group having from 1 to 22 carbon atoms, where one or more CH2 groups may be replaced by -0-, -S-, -CO-, -OCO-, -CO-0-, -0-00-0-, -CR¹=CR², -C-C-, SiR³R⁴, C4-C10-aryldiyl, C4-C10-heteroaryldiyl, cyclohexylene, 20 -NR⁵-, where heteroatoms must not be directly bonded to one another, and where one or more H atoms may be replaced by F, Cl, Br; where R¹ to R⁵ are as defined in the formula (1) in claim 2;
c) a C4-C12-aryl or heteroaryl group which may be substituted by one or more radicals R, where R are identical or different and R as defined in the formula (1) in claim 2.

4. The process as claimed in one or more of the preceding claims, wherein the ratio of activated group an the aromatic to amine is 1:0.8-2, preferably 1:1-1.5.

5. The process as claimed in one or more of the preceding claims, wherein a base selected from the group consisting of alkali metal and alkaline earth metal hydroxides, alkali metal and alkaline earth metal carbonates, alkali metal hydrogencarbonates, alkali metal and alkaline earth metal acetates, alkali metal and alkaline earth metal alkoxides and primary, secondary and tertiary amines is used.

6. The process as claimed in one or more of the preceding claims, wherein the palladium component used is palladium metal, a palladium compound or a palladium complex containing a phosphine ligand.

7. The process as claimed in one or more of the preceding claims, wherein the palladium component is used in an amount of from 0.01 to 10 mol%, preferably from 0.05 to 5 mol%, particularly preferably from 0.1 to 3 mol%, very particularly preferably from 0.1 to 1.5 mol%, based on N-H groups present.

8. The process as claimed in one or more of the preceding claims, wherein a phosphine ligand selected from the group consisting of trialkylphosphines, tricycloalkylphosphines and triarylphosphines, where the three substituents on the phosphorus may be identical or different, chiral or achiral and one or more of the ligands may link the phosphorus groups of a plurality of phosphines, where part of this linkage may also be one or more metal atoms, is used.

## Revendications

1. Procédé de production d'aryloligoamines qui contiennent au moins deux motifs amine liés à des groupes aromatiques, **caractérisé en ce que** l'on fait réagir une amine avec un composé aromatique activé de formule générale (I)
(R)ₙ-A-(X)ₘ (I)
dans laquelle les symboles et indices ont la signification suivante :
A est un radical aromatique avec 2 à 200 atomes de carbone, qui peut contenir plusieurs groupes aromatiques, de tels groupes pouvant être condensés ;
R sont, identiques ou différents, NO₂, CN, F, un groupe alkyle non ramifié ou ramifié avec 1 à 22 atomes de carbone, un ou plusieurs groupes CH₂ pouvant être remplacés par -O-, -S-, -CO-, -O-CO-, -CO-O-, -O-CO-O-, -CR¹=CR²-, -C≡C-, SiR³R⁴, un aryldiyle en C₄ à C₁₀, hétéroaryldiyle en C₄ à C₁₀, cyclohexylène, -NR⁵-, les hétéroatomes ne devant pas être liés directement les uns aux autres, et un ou plusieurs atomes H pouvant être remplacés par F, Cl, Br ;
R¹, R² sont, identiques ou différents, H, CN, un alkyle en C₁ à C₁₂, aryle en C₄ à C₁₀ ;
R³, R⁴ sont, identiques ou différents, un alkyle en C₁ à C₁₂, aryle en C₄ à C₁₀ ;
R⁵ est un alkyle en C₁ à C₁₂, aryle en C₄ à C₁₀ ;
X est Cl, Br, I, un mésylate, tosylate ou perfluoroalkylsulfonate en C₁ à C₁₂ ;
m est un nombre naturel, dans lequel 2 ≤ m ≤ y ;
n est un nombre naturel, dans lequel 0 ≤ n ≤ y-m ;
y est le nombre de valences libres sur le squelette A,
et une base dans une gamme de températures de 0 à 150 °C en présence d'un composant du palladium et d'un ligand phosphane, à l'exception du trialkylphosphane et du tricyclohexylphosphane.

2. Procédé de production d'aryloligoamines qui contiennent au moins deux motifs amine liés à des groupes aromatiques, **caractérisé en ce que** l'on fait réagir une amine avec un composé aromatique activé de formule (Ia) 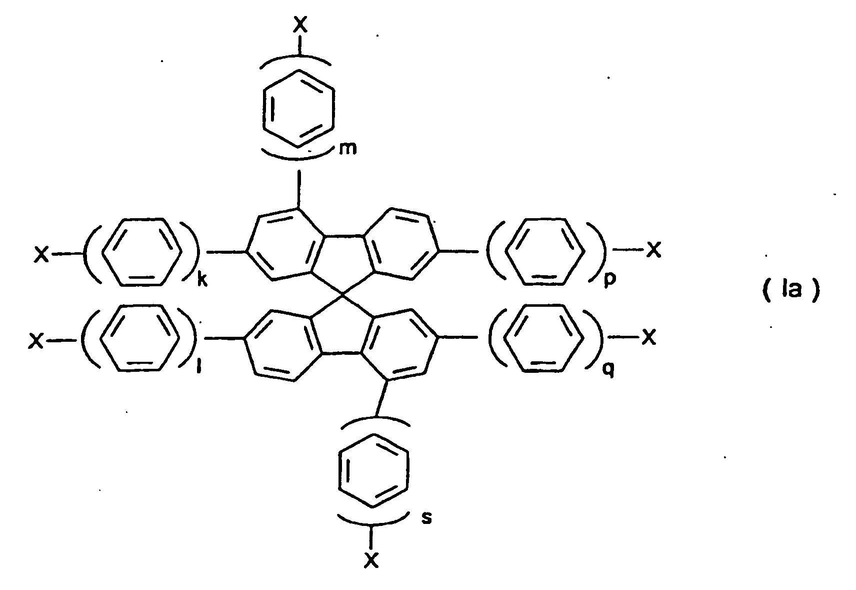 dans laquelle
X représentent, identiques ou différents, Br, I ou H et
k, l, p, q, m, s représentent 0, 1, 2, 3, 4, à condition qu'au moins deux des radicaux X soient Br ou I,
et une base dans une gamme de températures de 0 à 150 °C en présence d'un composant du palladium et d'un ligand phosphane.

3. Procédé selon une ou plusieurs des revendications précédentes, **caractérisé en ce qu'**on utilise un composant amine de formule (II),
H-NR⁷R⁸ (II)
dans laquelle les symboles ont les significations suivantes :
R⁷, R⁸ sont, identiques ou différents,
a) H ;
b) un groupe alkyle à chaîne linéaire, ramifié ou cyclique avec 1 à 22 atomes de carbone, un ou plusieurs groupes -CH₂- pouvant être remplacés par -O-, -S-, -CO-, -O-CO-, -CO-O-, -O-CO-O-, -CR¹=CR²-, -C≡C-, SiR³R⁴, un aryldiyle en C₄ à C₁₀, hétéroaryldiyle en C₄ à C₁₀, cyclohexylène, -NR⁵-, les hétéroatomes ne devant pas être liés directement les uns aux autres, et un ou plusieurs atomes H pouvant être remplacés par F, Cl, Br ; R¹ à R⁵ ayant les significations indiquées dans la formule (I) de la revendication 1 ;
c) un groupe aryle ou hétéroaryle en C₄ à C₁₂ qui peut être substitué par un ou plusieurs radicaux R, dans lequel les radicaux R, identiques ou différents, ont les significations indiquées dans la formule (I) de la revendication 1.

4. Procédé selon une ou plusieurs des revendications précédentes, **caractérisé en ce qu'**on choisit un rapport des groupes activés sur les composés aromatiques par rapport à l'amine de 1 : 0,8 à 2, de préférence de 1 : 1 à 1,5.

5. Procédé selon une ou plusieurs des revendications précédentes, **caractérisé en ce qu'**on utilise une base du groupe des hydroxydes de métaux alcalins et alcalino-terreux, des carbonates de métaux alcalins et alcalino-terreux, des hydrogénocarbonates de métaux alcalins, des acétates de métaux alcalins et alcalino-terreux, des alcoolates de métaux alcalins et alcalino-terreux et des amines primaires, secondaires et tertiaires.

6. Procédé selon une ou plusieurs des revendications précédentes, **caractérisé en ce qu'**on utilise comme composant du palladium le palladium métal, un composé du palladium ou un complexe de palladium, qui contient un ligand phosphane.

7. Procédé selon une ou plusieurs des revendications précédentes, **caractérisé en ce qu'**on utilise le composant de palladium dans une proportion de 0,01 à 10 % en moles, de préférence de 0,05 à 5 % en moles, de manière particulièrement préférée de 0,1 à 3 % en moles, en particulier de préférence de 0,1 à 1,5 % en moles, par rapport aux groupes NH présents.

8. Procédé selon une ou plusieurs des revendications précédentes, **caractérisé en ce qu'**on utilise un ligand phosphane du groupe des tricycloalkylphosphanes, triarylphosphanes, les trois substituants sur le phosphore pouvant être identiques ou différents, chiraux ou achiraux et un ou plusieurs des ligands pouvant relier les groupes phosphore de plusieurs phosphanes et une partie de ce couplage pouvant être aussi un ou plusieurs atomes de métal.
